# EUROPEAN PATENT SPECIFICATION

(11) **EP 1 797 175 B1**
(45) Date of publication and mention of the grant of the patent: **16.07.2008**
(21) Application number: 05798981.6
(22) Date of filing: 04.10.2005
(51) Int. Cl.: C12N 7/06, A61K 39/21

(54) **SUBTYPE-MATCHED INACTIVATED WHOLE VIRUS VACCINES FOR TREATING PATIENTS WITH HIV INFECTION**
SUBTYPABGESTIMMTE INAKTIVIERTE VOLLVIRUSVAKZINE ZUR BEHANDLUNG VON PATIENTEN MIT HIV-INFEKTION
VACCINS DE VIRUS ENTIERS INACTIVES CORRESPONDANT A UN SOUS-TYPE POUR TRAITER DES PATIENTS PRESENTANT UNE INFECTION VIH

(30) Priority: 04.10.2004 US 615729 P; 04.10.2005 US 243094
(43) Date of publication of application: 20.06.2007
(73) Proprietor: Biovaxim Limited, 5-7 Cranwood Street London EC1V 9EE (GB)
(72) Inventor: ANDRIEU, Jean-Marie, F-75005 Paris (FR); WEI-LU, Louis, F-75015 Paris (FR)
(74) Representative: Breese, Pierre
(86) International application number: PCT/IB2005/003384
(87) International publication number: WO 2006/038124

(56) References cited:
- WO-A-02/088328
- US-A1- 2004 009 194
- US-A1- 2004 109 876
- LAPENTA CATERINA ET AL: "Potent immune response against HIV-1 and protection from virus challenge in hu-PBL-SCID mice immunized with inactivated virus-pulsed dendritic cells generated in the presence of IFN-alpha." THE JOURNAL OF EXPERIMENTAL MEDICINE, vol. 198, no. 2, 21 July 2003 (2003-07-21), pages 361-367, XP002366695 ISSN: 0022-1007 cited in the application
- YOSHIDA ATSUSHI ET AL: "Induction of protective immune responses against R5 human immunodeficiency virus type 1 (HIV-1) infection in hu-PBL-SCID mice by intrasplenic immunization with HIV-1-pulsed dendritic cells: possible involvement of a novel factor of human CD4(+) T-cell origin." JOURNAL OF VIROLOGY, vol. 77, no. 16, August 2003 (2003-08), pages 8719-8728, XP002366696 ISSN: 0022-538X cited in the application
- LU WEI ET AL: "Therapeutic dendritic-cell vaccine for simian AIDS." NATURE MEDICINE, vol. 9, no. 1, January 2003 (2003-01), pages 27-32, XP002366697 ISSN: 1078-8956 cited in the application
- BHARDWAJ NINA ET AL: "Immunotherapy for AIDS virus infections: cautious optimism for cell-based vaccine." NATURE MEDICINE, vol. 9, no. 1, January 2003 (2003-01), pages 13-14, XP002366698 ISSN: 1078-8956
- LU WEI ET AL: "Therapeutic dendritic-cell vaccine for chronic HIV-1 infection." NATURE MEDICINE, vol. 10, no. 12, December 2004 (2004-12), pages 1359-1365, XP002366699 ISSN: 1078-8956

## Description

### Field of the Invention

This invention relates to the field of vaccines for the treatment of viral infection, in particular for the treatment of HIV infection in humans.

### Background

Twenty years after the discovery of human immunodeficiency virus (HIV), no effective preventive or therapeutic vaccine is so far available. Recent projections from the World Health Organization and the Joint United Nations Program on HIV/AIDS indicate that if the pandemic progresses at its current rate, there will be 45 million new infections by 2010 ¹. Although significant progress has been achieved in extending the survival of HIV-infected people and in reducing maternal-newborn HIV transmission by antiretroviral therapy, there are an increasing number of patients who develop drug resistance and/or severe drug-related adverse effects under long-term antiretroviral therapy. Additional alternative therapeutic strategies are thus urgently needed to protect HIV-infected individuals from disease progression.

In a large-scale human phase III trial, one candidate vaccine aimed at eliciting humoral immunity to neutralize HIV has recently failed to demonstrate its efficacy ². This lack of protection is inkeeping with the well-known inability of current vaccine designs to elicit effective neutralizing antibodies (Nab) against HIV in vivo ³. Such an inability to raise efficient Nab is probably determined by the nature of the infectious agent. For example, there is so far no successfully preventative vaccine for chronic infections such as tuberculosis, leprosy, and hepatitis C virus infection. In contrast, successful vaccines prevent acute infectious diseases such as polio, measles, diphtheria, tetanus, or small pox through the induction of Nab that can be also transferred transplacentally or via milk to protect foetus or newborn from infections.

It is well recognized that long-lasting cellular immunity can potentially control disease in situations where the infectious agent is not eradicated, such as in chronic infections with tuberculosis, leprosy, hepatitis B or C virus, and HIV. In this regard, vigorous virus-specific CD4⁺ T-helper type 1 (Thl)-cell and effector (perforin⁺) cytotoxic T lymphocytes (CTL) responses were shown to be associated with control of viremia and long-term nonprogression in individuals with chronic HIV-1 infection ⁴⁻¹⁰. In addition, early intervention with highly active antiretroviral therapy (HAART) during or shortly after acute infection was associated with enhanced HIV-1-specific CD4⁺ Th1-cell responses ^{11,12}. In contrast, at a later stage, HAART led to the decline of HIV-1-specific CD4⁺ Th1-cell and CTL responses ^{5,13,14}, suggesting that the functional capacities of HIV-1-capturing antigen presentation cells (APCs) (which are required for the induction of the immune response) are progressively lost along the course of the infection ¹⁵⁻¹⁸. In this context, a therapeutic vaccine aimed at eliciting HIV-specific long-lasting cellular immune responses could be feasible under the two prerequisite conditions: 1) the discovery of an appropriate immunogen capable of eliciting a strong, broad, and sustained protective cellular immunity; and 2) the reconstitution of in vivo impaired APC's function either by the adoptive transfer of ex vivo-activated APC (i.e. replacement strategy) or by a direct in vivo activation of less impaired DCs (such as Langerhans cells) by a simultaneous combination of the immunogen and appropriate cytokines or adjuvants. The ultimate goal of a successful therapeutic vaccine is to sustainably reduce the viral load of HIV-1-infected patients to as a low level as possible. This would protect them from disease progression, and thus reduce the requirement for harmful and expensive antiretroviral drugs. Moreover, sustainably reducing HIV viral load could minimize their risk of sexually transmitting the virus to healthy people ¹⁹.

A vaccine-induced protective immune response can differ substantially depending on the nature of the immunogen. Live-attenuated vaccines elicit both humoral and cellular immune responses, while killed virus vaccines and purified synthetic proteins preferentially elicit humoral (antibody) response. In a multi-center clinical trial aimed at triggering the cellular arm of the immune system, researchers were disappointed in that only 20% of 205 volunteers immunized with an experimental vaccine made of bacterial DNA containing HIV genes had a significant HIV-specific cellular immune response (although the DNA prime does work well in mouse experiments) ²⁰. In this context, the present inventors (and others) have recently discovered that inactivated whole HIV-1, in which the conformational structure of envelope protein gp120 is conserved (which is for example the case when the virus is treated by aldrithiol-2 [AT-2]), can be processed and presented by dendritic cells (DCs, the most potent APC) for inducing a potent HLA-I-restricted CTL response in vitro ^{21,22}. Several studies have also demonstrated that the adoptive transfer of autologous DCs loaded *in vitro* with inactivated whole HIV-1 induced protective antiviral immunity in hu-PBL-SCID mice ^{23,24}. The present inventors had previously shown that a therapeutic vaccine made of inactivated whole simian immunodeficiency virus (SIV) strain mac251 (SIVmac251)-loaded DCs led, in the absence of any other antiviral therapy, to dramatic viral suppression in Chinese rhesus monkeys immunized two months been infection with SIVmac251²⁵. Taken together, these findings suggest that AT-inactivated whole virus could be used as an efficient vaccine immunogen for eliciting protective HIV-1-specific CTL responses in people with chronic HIV-1 infection. However, the extensive global variability of HIV-1 argues against the concept of pharmaceutical use of a GMP-grade inactivated whole virus preparation as a universal therapeutic vaccine for HIV-1.
Lifson JD et al. (Aids Research and Human Retroviruses 20(7):772-787, July 2004) discloses the use of AT-2 inactivated whole cell-free SIV vaccine which is administered intravenously to seronegative macaques.

### Summary of the Invention

The invention provides the use of whole HIV of a specific subtype inactivated by aldithriol-2 (AT-2) treatment for producing a cell-free vaccine for treating an individual chronically infected with HIV by intradermic administration, wherein the vaccine induces a protective cellular immune response in the individual against the same HIV subtype used to produce the vaccine. The scope of the invention is defined in the claims.

### Brief Description of the Figure

Fig. 1 shows the cytotoxic T-lymphocyte (CTL) killing activity of vaccines of the invention.

Fig. 2 shows the effects of intra-dermal immunization with AT-2 inactivated SIV mac251 on the plasma viral load of chronically SIVmac251-infected macaques. The data represent the geometric mean of SIV RNA copies per milliliter of plasma before, and after immunization. P value represents the statistical analysis of paired data before, and at month 3 or 6 after the immunization by the Wilcoxon test.

### Detailed Description

There are two major HIV groups (HIV-1 and HIV-2) and many subgroups because the HIV genome mutates constantly. The major difference between the groups and subgroups is in the viral envelope. HIV-1 is classified into main subgroup (M) and a 10th outlier subgroup (O), in which subgroup M is divided into nine subtypes (clades) designed A through J ²⁸⁻²⁹. The genetic variation seen in the HIV genome is the result of mutation, recombination, insertion, and deletion ²⁹.

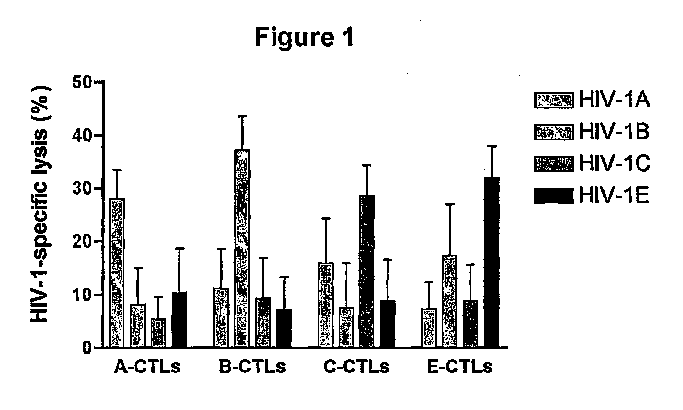

Vaccines against HIV, preferably HIV-1 subtypes like subtypes A, B, C, or E exhibited high CTL killing activity with subtype-matched viral strains while only low cross-subtype killing activity was observed. Although the cross-subtype killing activity was observed in individual cases, the CTL killing efficiency varied widely in the subtype-unmatched viral strains as compared to the subtype-matched viral strains (see Ex. 1 below and Fig. 1). Thus, herein disclosed is a subtype-specific therapeutic vaccine for HIV made with an inactivated whole HIV preparation of a given subtype. The vaccines can be used to treat an individual with a chronic HIV infection, wherein the vaccines induce a protective cellular immune response against the same HIV subtype used to produce the vaccine.

The term "vaccine" as used herein, refers to a composition that is administered to produce or artificially increase immunity to a particular disease.

Preferably, the vaccines are produced with an HIV subtype which is not autologous to the individual being treated. In fact, the inventors have shown that unexpectedly a vaccine with an HIV subtype which is not autologous to the individual to be treated enable to significantly decrease the viral charge of said individual.

A chronic infection with any HIV subtype can be treated, for example chronic infections with HIV virus is selected from one of the A, B, C, or E (and other) subtypes of the group M, as well HIV-2 group and HIV O subgroup.

The composition can comprise several inactivated HIV subtypes -e.g. two, three, four or more different inactivated HIV subtypes-.

As used herein, "treating" an individual with a chronic HIV infection means that symptoms of HIV infection are prevented, reduced or inhibited; viral load (in particular plasma viral load) is reduced after administering the vaccine; and/or an anti-HIV CTL response is induced in the individual. It is understood that "treating" an individual for chronic HIV infection does not require the complete eradication of HIV from the individual.

As used herein, "individual with a chronic HIV infection" means an individual, which can be diagnosed as infected by HIV.

As used herein, "inactivated whole HIV" means a complete HIV particle, which has been inactivated, and which is no more infectious.

In the practice, HIV of a specific subtype can be inactivated by any suitable technique known from one of skill in the art, such as ultraviolet irradiation, heat or chemical treatment, like formaldehyde, paraformaldehyde, propiolactene or AT-2 treatment.

According to the present invention, HIV of a specific subtype is inactivated by exposing the HIV to a chemical treatment, and more preferably to AT-2 treatment. Unexpectedly, such an AT-2 inactivation enables to maintain the conformational structure of the HIV proteins, and the induction of real potent CTL response with non autologous.

The subtype-specific inactivated whole virus immunogens can be used either for *ex vivo* loading (also called "pulsing") of antigen presenting immune cells (APCs), such as mature or immature dendritic cells (DCs) or Langerhans cells (LCs), to produce an APC-based therapeutic vaccine, or for a direct intradermic *in vivo* injection enabling the *in vivo* loading of LCs to produce a cell-free vaccine.

The cell-free vaccines of the invention are administered by, for example, direct (preferably needle-free) delivery of the inactivated subtype-specific HIV (*e.g*., by an intradermic injector) to patient's skin by methods within the skill in the art. Needle-free devices for intradermic vaccine administration are well known of one of skill in the art, and include as an example the devices described in patent US 6,933,319 and in International Patent Application WO 2004/101025. Vaccination through the skin (intradermal administration) is particularly advantageous, as the epidermis harbors large numbers of LCs. LCs are known to be the immature form of DCs which are located in close proximity to the most superficial layer of the skin, the stratum corneum. These LCs represent a network of immune cells that underlie 25% of the skin's surface area ²⁶ and remain functionally intact during the early or asymptomatic phase of chronic HIV/SIV infection ²⁷. The inventors have shown that such an intradermic administration of the cell-free vaccines of the invention enable to significantly decrease SIV viral charge.

In one embodiment the method comprises a previous step of determining the subtype of HIV infecting the patient to be treated before the administration of the composition of the invention. Such a determination can be done by methods well known in the art like genotyping of specific area of HIV sequence by analysis of HIV present in a blood sample from said patient. Preferably, this HIV subtype determination step is followed by the administration of APC-based or cell free vaccines of the invention comprising the same HIV subtype than those infecting the patient to be treated.

An individual may be treated with APC loaded with inactivated HIV of a specific subtype. The APC is first loaded with the inactivated HIV *ex vivo*, and the loaded APC is then administered to the patient by any suitable technique. The loaded APC is injected subcutaneously, intradermally or intramuscularly into the individual. More preferably, the APC are obtained by a previous PBMC sampling from the individual to be treated in order to isolate the monocytes (CD14+), which are then transformed with known cytokines in immature and then mature dendritic cells. Such methods are well known from one of skill in the art, as an example, such a method is described in example 1.

Unlike bacterial products, inactivated whole HIV alone are not as efficient for inducing maturation of DCs ²⁵ to allow an efficient migration of these cells to the draining lymph nodes, where they execute their immunostimulatory function. Therefore, a class of potent molecules known as adjuvants is preferably combined with the inactivated whole HIV for triggering optimal maturation of immune cells such as LCs, thereby allowing an efficient cellular immune response against HIV-1 to be generated.

The term "adjuvant" refers to a substance added to a vaccine to improve the immune response.

Suitable adjuvants include complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions, keyhole limpet hemocyanins, dinitrophenol, conventional bacterial products (such as cholera toxin, heat-labile enterotoxin, attenuated or killed BCG (bacille Calmette-Guerin) and Corynebacterium parvum, or BCG derived proteins), biochemical molecules (such as TNF-alpha, IL-1-beta, IL-6, PGE₂, or CD40L), or oligodeoxynucleotides containing a CpG motif. Examples of materials suitable for use in vaccine compositions are disclosed, e.g., in Osol, A., ed., Remington's Pharmaceutical Sciences, Mack Publishing Co., Easton, Pa. (1980), pp. 1324-1341.

Dendritic cells have a pivotal role in monocytes differentiation regulation, and more especially in the regulation of CD4-Th1 profile versus CD4-Th2 profile differentiation. Preferably, the cell-free vaccine composition of the invention comprises adjuvants able to stimulate dendritic cells in order to inhibit cellular differentiation in CD4-Th2 profile, which is often induced in chronic infection, and simultaneously able to stimulate dendritic cells in order to activate cellular differentiation in CD4-Th1 profile. Such adjuvants are well known of one of skill in the art, and includes bacterial products (such as some BCG-derived proteins like Ag85B) or chemical compounds like compounds with anti-COX activity, and more especially with anti-COX2 activity (such as VIOX®, CELEBREX® or RIBAVERIN®).

The vaccines disclosed herein can be formulated into pharmaceutical compositions (also called "medicaments") for treating an individual chronically infected with HIV. Pharmaceutical compositions of the invention are preferably sterile and pyrogen free, and also comprise a pharmaceutically acceptable carrier. Suitable pharmaceutically acceptable carriers include water, saline solutions (*e.g.,* physiological saline), viscosity adjusters and other conventional pharmaceutical excipients and/or additives used in the formulation of pharmaceutical compositions for use in humans. Suitable pharmaceutical excipients include stabilizers, antioxidants, osmolality adjusting agents, buffers, and pH adjusting agents. Suitable additives include physiologically biocompatible buffers (*e.g*., tromethamine hydrochloride and the like), chelants *(e*.*g*., DTPA, DTPA-bisamide and the like) or calcium chelate complexes (*e*.*g*., calcium DTPA, CaNaDTPA-bisamide and the like), or, optionally, additions of calcium or sodium salts (*e.g*., calcium chloride, calcium ascorbate, calcium gluconate, calcium lactate and the like). Formulation of pharmaceutical compositions are within the skill in the art, for example as described in Remington's Pharmaceutical Science, 17th ed., Mack Publishing Company, Easton, Pa. (1985).

A typical regimen for treating an individual chronically infected with HIV which can be alleviated by a cellular immune response by active therapy, comprises administration of an effective amount of a vaccine composition as described above, administered as a single treatment, or repeated as enhancing or booster dosages, over a period up to and including one week to about 24 months.

An "effective amount" of a vaccine composition is one which is sufficient to achieve a desired biological effect, in this case at least one of cellular or humoral immune response to HIV, preferably one cellular immune response. It is understood that the effective dosage will be dependent upon the age, sex, health, and weight of the recipient, kind of concurrent treatment, if any, frequency of treatment, and the nature of the effect desired. The ranges of effective doses provided below are not intended to limit the invention and represent preferred dose ranges. However, the most preferred dosage will be tailored to the individual subject, as is understood and determinable by one of skill in the art, without undue experimentation. See, e.g., Berkow (1987), infra, Goodman (1990), infra, Avery (1987), infra, Ebadi, Pharmacology, Little, Brown and Co., Boston, Mass. (1985), and Katsung (1992), infra.

Generally speaking, the dosage for a human adult will be from about 10⁶ -10¹⁴ inactivated whole HIV particles per dose, with 10⁸ -10¹² preferred. Whatever dosage is used, it should be a safe and effective amount as determined by known methods, as also described herein.

The invention will now be illustrated by the following non-limiting examples.

### EXAMPLES

### Example 1

### Methods

### Virus and cell samples

HIV-1 strains were obtained by CD8-depleted peripheral blood mononuclear cell (PBMC) culture from patients infected with HIV-1 subtypes A (n = 10), B (n = 10), C (n = 10), or E (n = 10). These HIV-1 subtype (A, B, C, or E) strains were then inactivated by AT-2 (Sigma, St Louis, Missouri) as described in ²¹. Monocyte-derived DCs were prepared from each patient by a standardized 7-day culture ²⁵ under GMP conditions. Briefly, freshly collected PBMCs were subjected to plastic adherence at a density of 10⁶ cells/cm² in the presence of 0.5% of clinical-use human serum albumin (LFB, Les Ulis, France). After 2-hour incubation at 37°C in 5% CO₂, non-adherent cells were removed by rinsing with sterile PBS buffer. Adherent cells were then cultured for 5 days in a complete medium containing clinical-grade CellGro DC medium (CellGenix, Freiburg, Germany) supplemented with 2000 U/ml GM-CSF (Schering-Plough, Brinny, Ireland) and 50 ng/ml clinical-grade IL-4 (CellGenix). At day 5, DCs were exposed to AT-2-inactivated autologous virus (10⁹ viral particles/ml) at 37°C for 2 h. After 2 washes to remove non-bound inactivated virus, cells were cultured for 2 additional days in the complete medium supplemented with clinical-grade cytokines IL-1β (10 ng/ml) (CellGenix), IL-6 (100 ng/ml) (CellGenix), and TNF-α (50 ng/ml) (CellGenix). At day 7, quality control (QC) of DCs was performed by flow cytometry ²⁵. QC-approved viable DCs were then used for expanding autologous virus-specific CTLs by a coculture protocol as described in ²¹, *supra*.

### Cytotoxic assay

DCs were pulsed with AT-HIV-1 (10⁹/ml) for 90 min then labeled with CFSE (Molecular Probes, PoortGebouw, The Netherlands) (10 nM) for 15 min and washed twice. Non-pulsed DCs were labeled with CFSE as specificity control. HIV-1 subtype strain-specific CTLs were plated into Micro Tubes-Bulk (Bio-Rad, Hercules, CA) in the presence of CFSE-labeled subtype-matched or unmatched AT-2-HIV-1-pulsed dendritic cells (DCs) at an E:T ratio of 10:1 for 4 h at 37°C. At the end of the incubation, 10 µl of propidium iodide (PI, Sigma) (20 µg/ml) were added to each tube. Target cytolysis was analyzed on a FACSCalibur (BD Immunocytometry System, San Jose, CA). Virus-specific cytolytic activity was determined by calculating the percentage of CFSE/PI-staining DCs after subtraction of the non-specific CFSE/PI-staining of non-pulsed DCs.

### Statistical analysis

Impaired data between HIV-1 subtype-matched and subtype-unmatched cell-killing activities were compared by the Mann-Whitney test.

### Results

Vaccines according to the present invention specific to HIV-1 subtypes A, B, C, or E exhibited high *in vitro* CTL killing activity (28-37%) with subtype-matched viral strains (n = 10), while only low (5-17%) cross-subtype killing activity was observed (P < 0.001). Although the cross-subtype killing activity was observed in individual cases, the CTL killing efficiency varied widely in the subtype-unmatched viral strains (SD/mean >50%) as compared to the subtype-matched viral strains (SD/mean <20%) (P < 0.001) (Fig. 1). These findings indicate that a pharmaceutical subtype-specific therapeutic vaccine for HIV-1 can be made by a GMP-grade inactivated whole virus preparation.

### Example 2

Individuals chronically infected with HIV are identified, and the HIV subtype with which the individual is infected is determined. Plasma viral load is determined for each individual prior to treatment with a vaccine of the invention. HIV of the same subtype as the subtype with which the individuals are infected is obtained, cultured and inactivated with AT-2 as described above in Example 1.

Inactivated HIV subtypes are administered to one group chronically infected individuals by needleless intradermal delivery, and plasma viral load is measured. It is expected that plasma viral load in the chronically infected individuals will be significantly reduced upon administration of the present vaccines.

Another group of chronically infected individuals are administered dendritic cells which have been loaded with HIV inactivated with AT-2 as described above in Example 1. The HIV-loaded dendritic cells are administered to the individuals. It is expected that plasma viral load in the chronically infected individuals will be significantly reduced upon administration of the loaded dendritic cells.

### Example 3

40 chronically (>1 year) SIVmac251-infected macaques with a plasma viral load > 1000 copies/ml were randomized to either receive a monthly intra-dermal injection (25 cm² on the back) of the SIVac LA2.1 (2.5 ml 0.9% NaCl solution containing 10¹⁰ AT-2-inactivated SIVmac251) for 5 months using an automated injecting pistol (AKRA DERMOJET, Pau, France) (100 µl/cm²/injection) (n = 20); or receive a monthly intra-dermal injection of placebo (2.5 ml 0.9% NaCl solution alone) for 5 months (n = 20). Plasma samples were collected from baseline and every month thereafter up to 6 months and stored at -80°C until use. Finally, the plasma SIV RNA load was measured by a quantitative RT-PCR assay (MUPROVAMA).

### Results

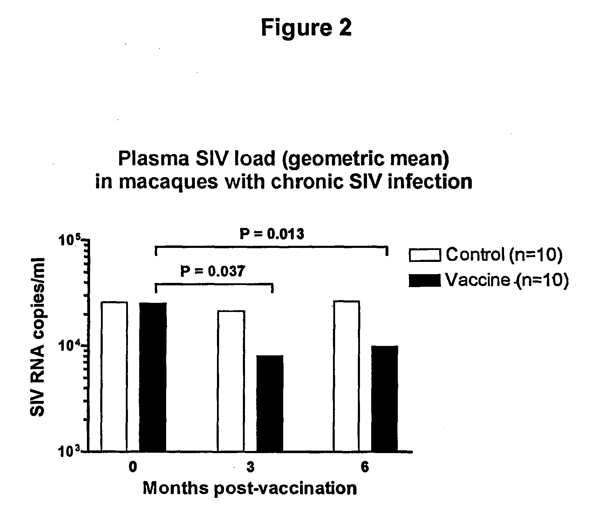

Cell-free vaccines as disclosed herein and specific to SIV subtype induce nearly 30% reduction (Fig. 2) in blood viral charge at 3 months (P=0.037), and at 6 months (P = 0.013). These findings indicate that a pharmaceutical subtype-specific therapeutic vaccine for SIV can be made by a GMP-grade inactivated whole virus preparation.

### Example 4

140 chronically (>1 year) SIVmac251-infected macaques with a plasma viral load > 1000 copies/ml were randomized to either receive a monthly intra-dermal injection (25 cm² on the back) of the SIVac LA2.1 (2.5 ml 0.9% NaCl solution containing 10¹⁰ AT-2-inactivated SIVmac251) with different adjuvants (10⁵ UFC of attenuated or heat-killed BCG, BCG-derived recombinant Ag85B) for 5 months using an automated injecting pistol (AKRA DERMOJET, Pau, France) (100 µl/cm²/injection) with or without a daily oral administration of 200 mg of CELEBREX ® for 4 weeks (n = 20 for each group); or receive a monthly intradermal injection of placebo (2.5 ml 0.9% NaCl solution alone) for 5 months (n = 20). Plasma samples were collected from baseline and every month thereafter up to 6 months and stored at -80°C until use. Finally, the plasma SIV RNA load was measured by a quantitative RT-PCR assay (MUPROVAMA).

### References

1. Stover, J. et al. Can we reverse the HIV/AIDS pandemic with an expanded response? Lancet 360, 73-7 (2002).
2. Cohen, J. HIV/AIDS. Vaccine results lose significance under scrutiny. Science 299, 1495 (2003).
3. Srivastava, I.K., Ulmer, J.B. & Barnett, S.W. Neutralizing antibody responses to HIV: role in protective immunity and challenges for vaccine design. Expert Rev Vaccines 3 Suppl 1, S33-52 (2004).
4. Rosenberg, E.S. et al. Vigorous HIV-1-specific CD4+ T cell responses associated with control of viremia. Science 278, 1447-50 (1997).
5. Pitcher, C.J. et al. HIV-1-specific CD4+ T cells are detectable in most individuals with active HIV-1 infection, but decline with prolonged viral suppression. Nat Med 5, 518-25 (1999).
6. Zaunders, J.J. et al. Identification of circulating antigen-specific CD4+ T lymphocytes with a CCR5+, cytotoxic phenotype in an HIV-1 long-term non-progressor and in CMV infection. Blood (2003).
7. Boaz, M.J., Waters, A., Murad, S., Easterbrook, P.J. & Vyakarnam, A. Presence of HIV-1 Gag-specific IFN-gamma+IL-2+ and CD28+IL-2+ CD4 T cell responses is associated with nonprogression in HIV-1 infection. J Immunol 169, 6376-85 (2002).
8. Younes, S.A. et al. HIV-1 viremia prevents the establishment of interleukin 2-producing HIV-specific memory CD4+ T cells endowed with proliferative capacity. J Exp Med 198, 1909-22 (2003).
9. Harari, A., Petitpierre, S., Vallelian, F. & Pantaleo, G. Skewed representation of functionally distinct populations of virus-specific CD4 T cells in HIV-1-infected subjects with progressive disease: changes after antiretroviral therapy. Blood 103, 966-72 (2004).
10. Hess, C. et al. HIV-1 specific CD8+T cells with an effector phenotype and control of viral replication. Lancet 363, 863-6 (2004).
11. Malhotra, U. et al. Effect of combination antiretroviral therapy on T-cell immunity in acute human immunodeficiency virus type 1 infection. J Infect Dis 181, 121-31 (2000).
12. Oxenius, A. et al. Early highly active antiretroviral therapy for acute HIV-1 infection preserves immune function of CD8+ and CD4+ T lymphocytes. Proc Natl Acad Sci U S A 97, 3382-7 (2000).
13. Gray, C.M. et al. Frequency of class I HLA-restricted anti-HIV CD8+T cells in individuals receiving highly active antiretroviral therapy (HAART). J Immunol 162, 1780-8. (1999).
14. Kalams, S.A. et al. Levels of human immunodeficiency virus type 1-specific cytotoxic T- lymphocyte effector and memory responses decline after suppression of viremia with highly active antiretroviral therapy. J Virol 73, 6721-8. (1999).
15. McIlroy, D. et al. Low CD83, but normal MHC class II and costimulatory molecule expression, on spleen dendritic cells from HIV+ patients. AIDS Res Hum Retroviruses 14, 505-13. (1998).
16. Grassi, F. et al. Depletion in blood CD11c-positive dendritic cells from HIV-infected patients. Aids 13, 759-66. (1999).
17. Donaghy, H. et al. Loss of blood CD11c(+) myeloid and CD11c(-) plasmacytoid dendritic cells in patients with HIV-1 infection correlates with HIV-1 RNA virus load. Blood 98, 2574-6. (2001).
18. Pacanowski, J. et al. Reduced blood CD123+ (lymphoid) and CD1 lc+ (myeloid) dendritic cell numbers in primary HIV-1 infection. Blood 98, 3016-21. (2001).
19. Quinn, T.C. et al. Viral load and heterosexual transmission of human immunodeficiency virus type 1. Rakai Project Study Group. N Engl J Med 342, 921-9 (2000).
20. Cohen, J. AIDS vaccines. HIV dodges one-two punch. Science 305, 1545-7 (2004).
21. Lu, W. & Andrieu, J.M. In vitro HIV eradication by autologous CD8+ T cells expanded with inactivated-virus-pulsed dendritic cells. J Virol 75, 8949-56 (2001).
22. Buseyne, F. et al. MHC-I-restricted presentation of HIV-1 virion antigens without viral replication. Nat Med 7, 344-9. (2001).
23. Lapenta, C. et al. Potent immune response against HIV-1 and protection from virus challenge in hu-PBL-SCID mice immunized with inactivated virus-pulsed dendritic cells generated in the presence of IFN-alpha. J Exp Med 198, 361-7 (2003).
24. Yoshida, A. et al. Induction of protective immune responses against R5 human immunodeficiency virus type 1 (HIV-1) infection in hu-PBL-SCID mice by intrasplenic immunization with HIV-1-pulsed dendritic cells: possible involvement of a novel factor of human CD4(+) T-cell origin. J Virol 77, 8719-28 (2003).
25. Lu, W., Wu, X., Lu, Y., Guo, W. & Andrieu, J.M. Therapeutic dendritic-cell vaccine for simian AIDS. Nat Med 9, 27-32 (2003).
26. Yu, R.C., Abrams, D.C., Alaibac, M. & Chu, A.C. Morphological and quantitative analyses of normal epidermal Langerhans cells using confocal scanning laser microscopy. Br J Dermatol 131, 843-8 (1994).
27. Zimmer, M.I. et al. Disrupted homeostasis of Langerhans cells and interdigitating dendritic cells in monkeys with AIDS. Blood 99, 2859-68. (2002).
28. Hu et al., JAMA 275:210-216. (1996).
29. Korber et al., Science 280:1868-1871. (1998).

## Claims

1. The use of whole HIV of a specific subtype inactivated by aldithriol-2 (AT-2) treatment for producing a cell-free vaccine for treating an individual chronically infected with HIV by intradermic administration, wherein the vaccine induces a protective cellular immune response in the individual against the same HIV subtype used to produce the vaccine.

2. The use of claim 1, wherein the inactivated whole HIV of a specific subtype is not autologous to the individual being treated for chronic HIV infection.

3. The use of claim 1, wherein the inactivated whole HIV of a specific subtype-is selected from group M subtypes, HIV-2 group and HIV O subgroup.

4. The use of claim 3, wherein the group M subtype is A, B, C or E.

5. The use of claim 1, further comprising an adjuvant.

6. The use of claim 5, wherein the adjuvant stimulates maturation of dendritic cells.

7. The use of claim 5, wherein the adjuvant is able to stimulate dendritic cells in order to inhibit cellular differentiation in CD4-Th2 profile.

8. The use of claim 7, wherein the adjuvant is able to stimulate dendritic cells in order to activate cellular differentiation in CD4-Th1 profile.

9. The use of claim 6, wherein the dendritic cell is a Langerhans cell.

## Patentansprüche

1. Verwendung von Ganz-HIV eines spezifischen Subtyps, der mittels Aldithriol-2-Behandlung (AT-2-Behandlung) inaktiviert wird, zum Herstellen eines zellfreien Vakzins zum Behandeln einer Einzelperson, die chronisch mit HIV infiziert ist, mittels intradermaler Verabreichung, wobei das Vakzin eine schützende zelluläre Immunantwort in der Einzelperson gegen denselben HIV-Subtyp induziert, der zum Herstellen des Vakzins verwendet wird.

2. Verwendung nach Anspruch 1, wobei das inaktivierte Ganz-HIV eines spezifischen Subtyps nicht zu der Einzelperson autolog ist, die auf chronische HIV-Infektion behandelt wird.

3. Verwendung nach Anspruch 1, wobei das inaktivierte Ganz-HIV eines spezifischen Subtyps aus Gruppe-M-Subtypen, der HIV-2-Gruppe und der HIV-O-Subgruppe ausgewählt ist.

4. Verwendung nach Anspruch 3, wobei der Gruppe-M-Subtyp A, B, C oder E ist.

5. Verwendung nach Anspruch 1, die weiterhin ein Adjuvans umfasst.

6. Verwendung nach Anspruch 5, wobei das Adjuvans die Reifung von dendritischen Retikulumzellen stimuliert.

7. Verwendung nach Anspruch 5, wobei das Adjuvans dendritische Retikulumzellen stimulieren kann, um Zelldifferenzierung im CD4-Th2-Profil zu inhibieren.

8. Verwendung nach Anspruch 7, wobei das Adjuvans dendritische Retikulumzellen stimulieren kann, um Zelldifferenzierung im CD4-Th1-Profil zu inhibieren.

9. Verwendung nach Anspruch 6, wobei die dendritische Retikulumzelle eine Langerhans'-Zelle ist.

## Revendications

1. Utilisation de VIH entier d'un sous-type spécifique inactivé par un traitement à l'aldithriol-2 (AT-2) pour la production d'un vaccin acellulaire destiné au traitement d'un individu infecté de manière chronique par le VIH par une administration intradermique, dans laquelle le vaccin induit une réponse immunitaire cellulaire protectrice chez l'individu contre le même sous-type du VIH utilisé pour produire le vaccin.

2. Utilisation selon la revendication 1, dans laquelle le VIH entier inactivé d'un sous-type spécifique n'est pas autologue à l'individu traité pour une infection chronique par le VIH.

3. Utilisation selon la revendication 1, dans laquelle le VIH entier inactivé d'un sous-type spécifique est choisi parmi les sous-types du groupe M, le groupe VIH-2 et le sous-groupe VIH O.

4. Utilisation selon la revendication 3, dans laquelle le sous-type du groupe M est A, B, C ou E.

5. Utilisation selon la revendication 1, comprenant en outre un adjuvant.

6. Utilisation selon la revendication 5, dans laquelle l'adjuvant stimule la maturation des cellules dendritiques.

7. Utilisation selon la revendication 5, dans laquelle l'adjuvant est capable de stimuler les cellules dendritiques afin d'inhiber la différenciation cellulaire dans le profil CD4-Th2.

8. Utilisation selon la revendication 7, dans laquelle l'adjuvant est capable de stimuler les cellules dendritiques afin d'activer la différenciation cellulaire dans le profil CD4-Th1.

9. Utilisation selon la revendication 6, dans laquelle la cellule dendritique est une cellule de Langerhans.
